# EUROPÄISCHE PATENTSCHRIFT

(11) **EP 3 079 765 B1**
(45) Veröffentlichungstag und Bekanntmachung des Hinweises auf die Patenterteilung: **18.07.2018**
(21) Anmeldenummer: 14831084.0
(22) Anmeldetag: 12.12.2014
(51) Int. Cl.: A61N 1/44, A61B 18/04, A61L 2/14

(54) **ANORDNUNG ZUR BEHANDLUNG VON WUNDEN**
ASSEMBLY FOR TREATING WOUNDS
DISPOSITIF POUR TRAITER DES PLAIES

(30) Priorität: 12.12.2013 DE 102013113941
(43) Veröffentlichungstag der Anmeldung: 19.10.2016
(73) Patentinhaber: Relyon Plasma GmbH, 93057 Regensburg (DE)
(72) Erfinder: NETTESHEIM, Stefan, 93051 Regensburg (DE); KORZEC, Dariusz, 93173 Wenzenbach (DE); BURGER, Dominik, 93087 Alteglofsheim (DE)
(74) Vertreter: Reichert & Lindner Partnerschaft Patentanwälte
(86) Internationale Anmeldenummer: PCT/IB2014/066837
(87) Internationale Veröffentlichungsnummer: WO 2015/087287

(56) Entgegenhaltungen:
- EP-A1- 2 170 022
- EP-B1- 0 957 793
- WO-A1-2012/150040
- WO-A2-2011/022069
- DE-A1-102010 015 899
- DE-U1-202008 008 980

## Beschreibung

Die Erfindung betrifft eine Anordnung zur Behandlung von Wunden. Im Besonderen umfasst die Anordnung zur Behandlung von Wunden ein Gerät zur Erzeugung eines Plasmas und/oder eines angeregten Gases bzw. Gasgemisches mit einem piezoelektrischen Transformator. Das Gerät weist ein Gehäuse auf, in dem der piezoelektrische Transformator zusammen mit einer Platine untergebracht ist. Auf der Platine ist eine Steuerschaltung realisiert, mit der der piezoelektrische Transformator elektrisch verbunden ist. Ein mit dem Gerät erzeugtes Plasma und/oder das angeregte Gas bzw. Gasgemisch tritt aus einer Öffnung des Gehäuses aus.

Die noch nicht veröffentlichte Patentanmeldung DE 10 2013 107 448.0 offenbart eine Vorrichtung zur Keimreduktion mittels Plasma. Die dielektrische Folie schließt mit ihrem umlaufenden Rand einen zu entkeimenden Bereich (Wunde oder Gegenstand) ein. Ein Hochspannungsende des piezoelektrischen Transformators ist der Außenseite der dielektrischen Folie zugewandt und das Plasma innerhalb der dielektrischen Folie gezündet.

Die deutsche Patentanmeldung DE 10 2012 103 362 A1 zeigt ein Plasma-Behandlungsgerät zur Behandlung des menschlichen Körpers. Das Plasma-Behandlungsgerät ist stiftartig ausgebildet. Hierzu ist eine erste Elektrode in einem Stiftteil vorgesehen. Das Stiftteil ist im Bereich der Elektrode geerdet. Gemäß einer Ausführungsform ist eine zweite Elektrode vorgesehen, die kreisförmig ist und auf die zu behandelnde Hautpartie aufgesetzt wird. Die zweite Elektrode dient zur Einstellung eines Abstandes. Gemäß einer zweiten Ausführungsform ist mit dem Erdungsleiter eine Erdungshülle verbunden. Hierdurch kann mittels der Erdungshülle auch ein konstanter Abstand zur Hautpartie eingestellt werden.

Die koreanische Patentanmeldung KR 2013-0023588 A betrifft ein Plasmaarray, das mit einem Anstandselement von der zu behandelnden Wunde beabstandet ist.

Das US-Patent US 8,267,884 B1 betrifft ein zylindrisches Gehäuse einer Plasmaeinrichtung. Eine Kappe kann mit der Düse der Plasmaeinrichtung verbunden werden. Die Kappe kann abgenommen und sterilisiert oder entsorgt werden.

Die internationale Patentanmeldung WO 02/32332 A1 offenbart eine Kammer, die in Kontakt mit der zu behandelnden Hautoberfläche ist. Mittels einer Vakuumpumpe wird das erzeugte und heiße Plasma wieder abgesaugt.

Die deutsche Patentanmeldung DE 10 2012 003 563 A1 offenbart eine Einrichtung zur desinfizierenden Wundbehandlung, mit einem Gehäuse, wobei in dem Gehäuse ein Plasmagenerator zum Erzeugen eines desinfizierenden Plasmas angeordnet ist. In dem Gehäuse ist ferner ein Strömungsmodul zum Erzeugen eines Gasstroms vorgesehen, der einen das desinfizierende Plasma aus dem Gehäuse transportierenden Freistrahl bildet. Eine Strahlsteuereinheit dient zur planmäßigen Beeinflussung des Freistrahls durch Steuern des von dem Strömungsmodul erzeugten Gasstroms. Gemäß einer Ausführungsform ist die Wunde von einer Schürze mit Absaugöffnungen umgeben und ein Absaugstutzen führt zu einer Absaugleitung.

Die deutsche Patentanmeldung DE 10 2007 054 161 A1 offenbart ein Verfahren zur Sterilisation von gestreckten Werkstücken. Mit dem hier beschriebenen Plasmabehandlungsverfahren wird eine Oberflächendekontamination erreicht, um Mikroorganismen und Viren mit einem Niedertemperaturplasma abzutöten. Das Verfahren zeichnet sich dadurch aus, dass dem Plasma verschiedene geeignete Zusätze beigemischt werden, um eine möglichst gute Abtötung der Mikroorganismen, bzw. Viren zu erreichen. Das Verfahren wird bei nicht organischen Körpern angewendet.

Die deutsche Patentanmeldung DE 10 2011 001 416 A1 offenbart eine Plasmabehandlungseinrichtung zur Behandlung von Wunden oder erkrankten Hautpartien. Die Plasmabehandlungseinrichtung weist zwei flexible Flächenelektroden zur Erzeugung eines nicht-thermischen Plasmas auf. Die beiden Flächenelektroden bestehen jeweils aus mindestens einem elektrischen Leiter, wobei die Leiter miteinander verwoben sind. Auf der der zu behandelnden Oberfläche zugewandten Außenseite der Flächenelektroden ist eine Wundkontaktschicht aus einem antiseptisch behandelten Material lösbar befestigt.

Die internationale Patentanmeldung WO 2010/034451 A1 offenbart einen Plasma-Applikator zum Anlegen eines nicht-thermischen Plasmas auf eine Oberfläche, insbesondere für die Plasmabehandlung von lebendem Gewebe und insbesondere für die Plasmabehandlung von Wunden. Der Plasma-Applikator umfasst einen Verschlussdeckel zum Abdecken eines Teils der Oberfläche. Dadurch wird ein Hohlraum zwischen dem Verschlussdeckel und der Oberfläche geschaffen. Das nicht-thermische Plasma ist im Hohlraum vorgesehen und zusätzlich kann der Hohlraum mit Gas gespült werden. Ebenso ist eine Pumpe vorgesehen, die Gas aus dem Hohlraum absaugt.

Die internationale Patentanmeldung WO 2012/158443 A2 offenbart eine Vorrichtung zur Erzeugung eines kalten Plasmas. Die Vorrichtung umfasst eine von Hand gehaltene Düse mittels der das Plasma zur Heilung von Wunden, zur Verbesserung von Anomalien der Hautoberfläche und zum Abtöten von Krankheitserregern auf die zu behandelnde Stelle gerichtet wird.

In der internationalen Patentanmeldung WO 2011/110342 A1 ist ein Plasma-Behandlungsgerät zum Aufbringen eines nicht-thermischen Plasmas durch ein Pflaster oder einen Verband offenbart, um eine Plasmabehandlung von Wunden zu erzielen. Das Plasma-Behandlungsgerät kann mit einer Abdeckung versehen werden. In diesem Fall wird das Plasma an der äußeren Oberfläche der Abdeckung erzeugt.

Die internationale Patentanmeldung WO 2013/076102 A1 offenbart eine Vorrichtung, mit der ein nicht-thermisches Plasma erzeugt wird. Eine Wandung umschließt den Reaktionsbereich und mittels der Wandung ist die Plasmaquelle auch von der Oberfläche beabstandet. Für den distalen Wandbereich wird elastisches Material verwendet, um eine Abdichtung des Reaktionsbereichs zu erreichen.

Die deutsche Patentanmeldung DE 10 2010 015 899 A1 offenbart eine elektrochirurgische Anordnung mit einem elektrochirurgischen Instrument und einem mit dem Instrument verbundenen Behandlungsgerät. In dem Behandlungsgerät ist ein Stromgenerator zur Bereitstellung von HF-Energie vorgesehen. Eine Ausführungsform des elektrochirurgischen Instruments hat gemäß einer Ausführungsform einen kegelförmig erweiternden Aufsatz. Durch eine Elektrode hindurch wird dem Behandlungsort ein Behandlungs-Fluid zugeführt. Bei einer Behandlung werden hier folglich diverse, separate Instrumente und Geräte verwendet.
Die europäische Patentschrift EP 0 957 793 B1 offenbart ein Endoskop, an dessen distalen Ende ein flexibles Hohlrohr angebracht ist. In dem Hohlrohr ist eine Elektrode angeordnet, die über eine Leitung mit einem Hochfrequenzgenerator und einem Argon Reservoir verbunden ist. Aus dem distalen Ende des Rohrs wird das ionisierte Plasma den Behandlungsort zugeführt. In einer Ausführungsform ist das distale Ende des Rohrs konisch geformt.
In der US-Patentanmeldung US 2012/0215158 A1 ist ein Endoskop offenbart, an das ein Kappenförmiger Aufsatz angebracht ist. In dem Aufsatz ist eine Plasma Entzündungsvorrichtung, mit der das zugeführte Plasma entzündet und dem Behandlungsort zugeführt werden kann.
Die deutsche Patentanmeldung DE 10 2009 041 167 A1 offenbart ein Multifunktionselement zur Durchführung von elektrochirurgischen Eingriffen. In dem Multifunktionselement ist eine Elektrode, die mit einem Hochfrequenzgenerator angeschlossen ist, der hochfrequenten Strom liefert. Gleichzeitig dient die Elektrode als Zufuhrkanal für Fluidkomponenten.
Das deutsche Gebrauchsmuster DE 20 2008 008 980 U1 offenbart eine Vorrichtung zur Erzeugung eines Atmosphärendruck-Plasmas. Ein Gehäuse weist eine dielektrische Endkappe auf, wobei in dem Gehäuse ein Piezoelement und eine Ansteuerplatine vorgesehen sind. Das Piezoelement endet im Bereich der Austrittsöffnung des Gehäuses. Dieses Gebrauchsmuster offenbart den nächstliegenden Stand der Technik. Der Erfindung liegt die Aufgabe zugrunde, eine Anordnung zur Behandlung von Wunden zu schaffen, die kostengünstig ist, einfach anzuwenden ist und mit der eine effiziente und sichere Behandlung von Wunden gegeben ist.

Die obige Aufgabe wird mit einer Anordnung gemäß den Merkmalen des Anspruchs 1 gelöst.

Die erfindungsgemäße Anordnung zur Behandlung von Wunden hat am Gerät zur Erzeugung eines Plasmas und/oder eines angeregten Gases bzw. Gasgemisches ein Expansionselement, mit einem ersten Ende derart lösbar befestigt, dass die Öffnung des Gehäuses des Geräts in das Expansionselement geführt ist. Mit einem zweiten Ende umgibt das Gerät einen zu behandelnden Wundbereich. Das Gerät hat eine Querschnittsfläche am zweiten Ende des Expansionselements ausgebildet, die größer ist als eine Querschnittsfläche des ersten Endes des Expansionselements.

Das Gerät kann ein Plasmagenerator oder lonisator oder Ozonisator sein. Für den Fall, dass das Gerät eine lonisator oder Ozonisator ist, wirkt anstelle eines Plasmas ein Gasgemisch mit angeregten Molekülen, Ionen und reaktiven Sauerstoffspezies, wie z.B. Ozon, atomarer Sauerstoff, H2O2, OH-Radikale oder NOx, auf den Wundbereich ein. Streng genommen muss nicht unbedingt ein Plasma den Wundbereich erreichen.

Die Wirkung auf der Oberfläche des Wundbereichs hängt von der lokalen Konzentration der aktiven Gasspezies ab. Da sich die aktiven Gasspezies an der Oberfläche des Wundbereichs abreagieren, muss hier ein durch die Strömungsverhältnisse begünstigter Gasaustausch erzwungen werden. Für die Wirkung ist nicht nur die integrale Dosis der aktiven Gasspezies entscheidend, sondern auch die maximale Konzentration an der Oberfläche des Wundbereichs. Eine kurz anliegende sehr hohe Konzentration ist wirkungsvoller als eine lang anliegende niedrige Konzentration. Eine kurze Exposition in Verbindung mit einer hohen Konzentration der Gasspezies resultieren in einer maximalen Oberflächenaktivität.

Eine Mantelfläche des Expansionselements hat mehrere Durchbrüche ausgebildet. Über die Durchbrüche kann ein Gasaustauch bzw. ein Druckausgleich zwischen dem Innern des Expansionselements und zumindest der Umgebung erfolgen. An einer Innenfläche der Mantelfläche ist mindestens ein Strömungsleitelement angebracht, damit eine Strömung aus Plasma oder angeregtem Gas bzw. Gasgemisch auf den zu behandelnden Wundbereich effizient gerichtet werden kann.

Eine Rückführung für Gas kann das Expansionselement und das Gerät miteinander verbinden. Ein Farbsensor kann ebenfalls dem Expansionselement zugeordnet sein, um über eine Farbänderung anzuzeigen, dass die Behandlung abgeschlossen ist. Ferner kann ein RFID-Chip dem Expansionselement zugeordnet sein, so dass über den Typ des Expansionselements und in Verbindung mit dem Gerät zumindest eine Behandlungsdauer einstellbar ist. Mittels des RFID-Chips ist es möglich, das Gerät derart abzustimmen, dass zumindest die Stärke und die Dauer des erzeugten Plasmas und/oder des angeregten Gases bzw. Gasgemisches, ggf. die Zuführung von zusätzlichem Gas (wie z.B. Argon), auf den Typ des Expansionselements abgestimmt sind.

Erfindungsgemäß ist das Gerät ein Plasmaerzeugungsgerät, das mit einem piezoelektrischen Transformator zur Erzeugung des Plasmas versehen ist. Das Hochspannungsende des piezoelektrischen Transformators ist zur Öffnung im Gehäuse hin ausgerichtet. Das Plasmaerzeugungsgerät ist als Handgerät ausgebildet.

Es ist von Vorteil, dass das Gerät ein Handgerät ist. Das Handgerät ist kostengünstig und gewährleistet eine einfache Handhabung. In Verbindung mit dem Expansionselement kann ein für die jeweilige Behandlung der Wunde erforderlicher Abstand, trotz Handgerät, eingehalten werden. Hinzu kommt, dass die Expansionselemente in einfacher und kostengünstiger Weise und mit unterschiedlicher Ausstattung hergestellt werden können.

Weitere Vorteile und vorteilhafte Ausgestaltungen der Erfindung sind Gegenstand der nachfolgenden Figuren, sowie deren Beschreibungsteile.

Es zeigen im Einzelnen:
- **Figur 1**: eine schematische Ansicht des prinzipiellen Aufbaus der erfindungsgemäßen Anordnung zur Plasmabehandlung von Wunden;
- **Figur 2**: eine perspektivische Ansicht einer Ausführungsform eines Expansionselements, das zusammen mit dem Plasmaerzeugungsgerät Anwendung findet;
- **Figur 3**: eine Draufsicht auf das Expansionselement;
- **Figur 4**: eine perspektivische Ansicht einer weiteren Ausführungsform des Expansionselements; und
- **Figur 5**: eine perspektivische Ansicht einer zusätzlichen Ausführungsform des Expansionselements.

Für gleiche oder gleich wirkende Elemente der Erfindung werden identische Bezugszeichen verwendet. Die dargestellten Ausführungsformen stellen lediglich eine Möglichkeit dar, wie die Anordnung zur Behandlung von Wunden mittels eines Plasmas ausgestaltet sein kann. Obwohl sich die nachstehende Beschreibung ausschließlich auf ein Gerät bezieht, das als Plasmaerzeugungsgerät ausgebildet ist, soll dies nicht als Beschränkung der Erfindung aufgefasst werden. Wie bereits oben erwähnt, kann anstelle eines Plasmas auch ein Gasgemisch mit angeregten Molekülen, Ionen und reaktiven Sauerstoffspezies, wie z.B. Ozon, atomarer Sauerstoff, H2O2, OH-Radikale oder NOx, die Wirkung bei der Behandlung von Wunden entfalten. Streng genommen muss auch nicht unbedingt Plasma den Wundbereich erreichen.

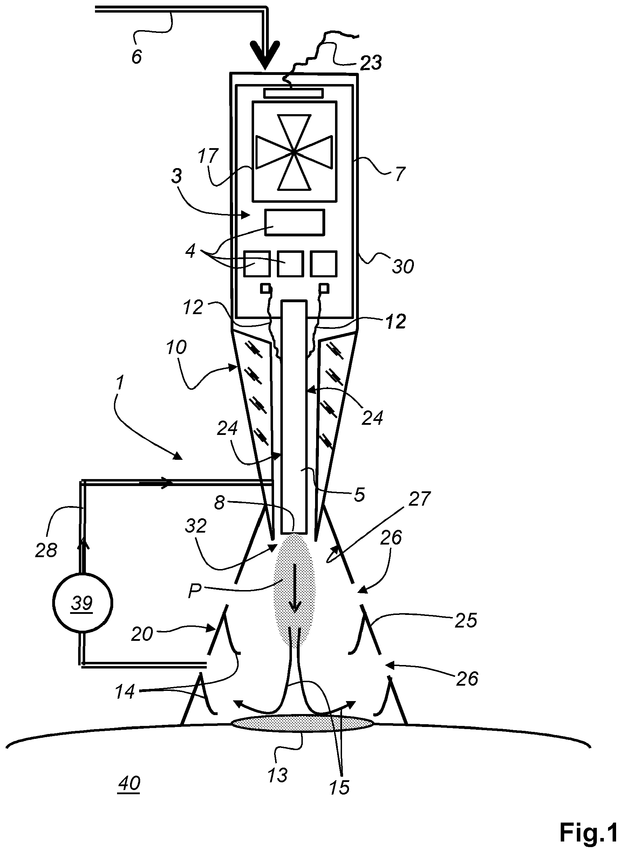

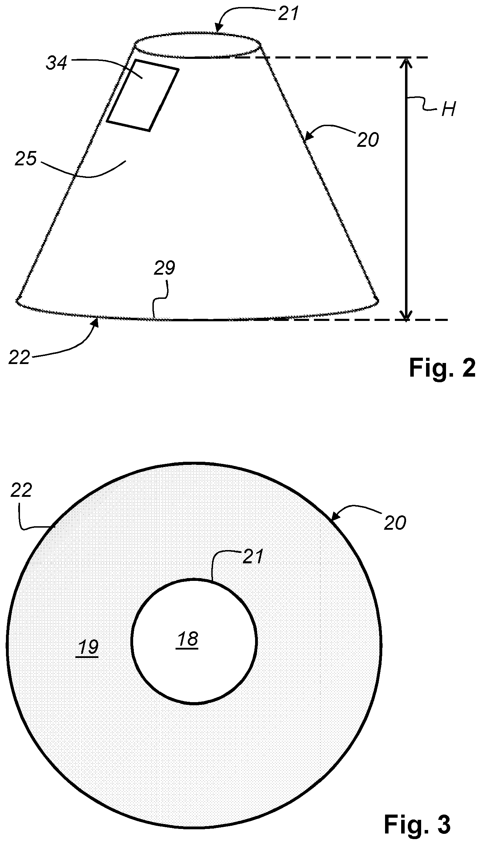

Eine schematische Ansicht der erfindungsgemäßen Anordnung 1 zur Behandlung von Wunden ist in **Figur 1** dargestellt. Bei der hier dargestellten Ausführungsform wird das Plasma P in einem Plasmaerzeugungsgerät 10 mit einem piezoelektrischen Transformator 5 erzeugt. Der piezoelektrische Transformator 5 ist in einem Gehäuse 30 des Plasmaerzeugungsgeräts 10 untergebracht. Es ist für einen Fachmann selbstverständlich, dass das Plasma P auch auf anderem Wege als mit einem piezoelektrischen Transformator 5 erzeugt werden kann. Obwohl sich die nachstehende Beschreibung auf einen piezoelektrischen Transformator 5 bezieht, soll die nicht als eine Beschränkung der Erfindung aufgefasst werden. Zur Ansteuerung ist der piezoelektrische Transformator 5 mit einer Platine 7 verbunden. Die Platine 7 realisiert mit einer Vielzahl von elektronischen Bauelementen 4 eine Steuerschaltung 3. Mit der Steuerschaltung 3 ist es möglich, den piezoelektrischen Transformator 5 mit seiner Resonanzfrequenz anzuregen. Die Steuerschaltung 3 für den piezoelektrischen Transformator 5 kann mit einer externen Energieversorgung verbunden werden, die ein herkömmliches Standard-Netzteil (nicht dargestellt) ist, das über ein Kabel 23 mit dem Gehäuse 30 des piezoelektrischen Transformators 5 verbunden ist. Ebenso kann die Energieversorgung mit einem Akku durchgeführt werden. Eine Kombination aus Akku und Standard-Netzteil ist ebenfalls denkbar. Die Ansteuerspannung wird von der Steuerschaltung 3 der Platine 7 über je einen elektrischen Anschluss 12 an je eine Seitenfläche 24 des piezoelektrischen Transformators 5 angelegt. Durch die an den Seitenflächen 24 des piezoelektrischen Transformators 5 anliegende Anregungsspannung bildet sich am Hochspannungsende 8 des piezoelektrischen Transformators 5 die erforderliche Hochspannung aus. Im oder an dem Gehäuse 30 ist ferner ein Lüfter 17 vorgesehen, der im Gehäuse 30 für eine Luftströmung zur Öffnung 32 des Gehäuses 30 hin sorgt. Ebenso kann über eine Gasleitung 6 dem Plasmaerzeugungsgerät 10 Gas oder ein Gasgemisch zugeführt werden, mit dem über dem piezoelektrischen Transformator 5 das Plasma gezündet wird.
Das Expansionselement 20 ist derart gestaltet, dass es mit einem ersten Ende 21 (siehe Figur 2) lösbar mit dem Gehäuse 30 des Plasmaerzeugungsgeräts 10 verbunden werden kann. Das zweite Ende 22 (siehe Figur 2) des Expansionselements 20 ist derart gestaltet, dass es mit einem umlaufenden Rand 29 (siehe Figur 2) des zweiten Endes 22 den zu behandelnden Wundbereich 13 umschließt. Ebenso sitzt das Expansionselement 20 mit dem umlaufenden Rand 29 auf dem Körperteil 40 auf, an dem sich der zu behandelnde Wundbereich 13 befindet. Damit sich eine Plasmaströmung 15 im Expansionselement 20 ausbreiten kann und für eine effektive Behandlung des Wundbereichs 13 sorgt, hat das zweite Ende 22 des Expansionselements 20 eine Querschnittsfläche 19 (siehe Figur 3) ausgebildet, die größer ist als eine Querschnittsfläche 18 (siehe Figur 3) am ersten Ende 21 des Expansionselements 20.

Um den Druck innerhalb des Expansionselements 20 in etwa auf dem Druckniveau des Umgebungsdrucks zu halten, hat eine Mantelfläche 25 des Expansionselements 20 mehrere Durchbrüche 26 ausgebildet. Über die Durchbrüche 26 können das überschüssige Reaktionsgas und die Reaktionsprodukte entweichen. An der Öffnung 32 des Gehäuses 30, also im Bereich des ersten Endes 21 des Expansionselementes 20, liegt eine hohe Strömungsgeschwindigkeit an. Hierdurch kann über den Venturi-Effekt bei den geeignet angeordneten Durchbrüchen 26 des Expansionselementes 20 Umgebungsgas (Luft) angesaugt werden.

Zur besseren Ausbildung einer Plasmaströmung 15 im Innern des Expansionselements 20 ist an einer Innenfläche 27 der Mantelfläche 25 mindestens ein Strömungsleitelement 14 angebracht. Die Plasmaströmung 15 soll so ausgebildet sein, dass sie auf den zu behandelnden Wundbereich 13 gerichtet ist und eine optimierte Einwirkzeit mit dem zu behandelnden Wundbereich 13 aufweist. Ebenso kann eine Rückführung 28 vorgesehen sein, die das Expansionselement 20 und das Plasmaerzeugungsgerät 10 miteinander verbindet. Gemäß einer weiteren Ausführungsform kann die Rückführung 28 noch mit einer Pumpe gekoppelt werden um eine aktive Re-Zirkulation zu erzwingen. Die Zirkulation kann bis hin zu einem Verhältnis von 10:1 des Eingangsvolumenstroms zum Zirkulationsvolumenstrom betragen. Über die Rückführung 28 kann somit Gas aus dem Innern des Expansionselements 20 gezielt dem Plasmaerzeugungsgerät 10 zugeführt werden. Bevorzugt wird das Gas in den Bereich des Hochspannungsendes 8 des piezoelektrischen Transformators 5 geleitet.

In **Figur 2** ist eine mögliche Ausführungsform des Expansionselements 20 dargestellt. Wie bereits in der Beschreibung zu Figur 1 erwähnt wir das Expansionselement 20 über sein erstes Ende 21 lösbar mit dem Plasmaerzeugungsgerät 10 (siehe Figur 1) verbunden. Obwohl sich die nachstehende Beschreibung auf ein Expansionselement 20 bezieht, das trichterförmig ausgebildet ist, soll die nicht als eine Beschränkung der Erfindung aufgefasst werden. Es ist für einen Fachmann selbstverständlich, dass die Form des Expansionselements 20 für die Verwendbarkeit bei der gegenwärtigen Erfindung nicht von Bedeutung ist.

Eine wichtige Bedingung für die Verwendung des Expansionselements 20 ist, wie in **Figur 3** dargestellt, dass das Expansionselement 20 an seinem ersten Ende 21 eine Querschnittsfläche 18 ausgebildet hat, die kleiner ist als eine Querschnittsfläche 19 des zweiten Endes 22 des Expansionselements 20. Ferner sollte der umlaufende Rand 29 des Expansionselements 20 am zweiten freien Ende 22 derart bemessen sein, dass er den zu behandelnden Wundbereich 13 umgibt. Zusätzlich sollte die Querschnittsfläche 18 am ersten Ende 21 des Expansionselements 20 der Form des Plasmaerzeugungsgeräts 10 im Bereich der Öffnung 32 des Gehäuses 30 entsprechen, damit das Expansionselement 20 lösbar und formschlüssig am Gehäuse 30 des Plasmaerzeugungsgeräts 10 befestigt werden kann. Ferner kann das Expansionselement 20 mit einem RFID-Chip 34 versehen werden, damit das Plasmaerzeugungsgerät 10 den Typ des Expansionselements 20 auslesen und über die Steuerschaltung entsprechende Einstellungen des Plasmaerzeugungsgeräts 10 vornehmen kann. Ferner kann über den RFID-Chip 34 auch eine Höhe H des Expansionselements 20 ausgelesen werden. Die Höhe H steht für Abstand des Plasmaerzeugungsgeräts 10 zu dem zu behandelnden Wundbereich 13. Auch die Höhe H stellt einen Einstellungsparameter für das Plasmaerzeugungsgerät 10 dar.

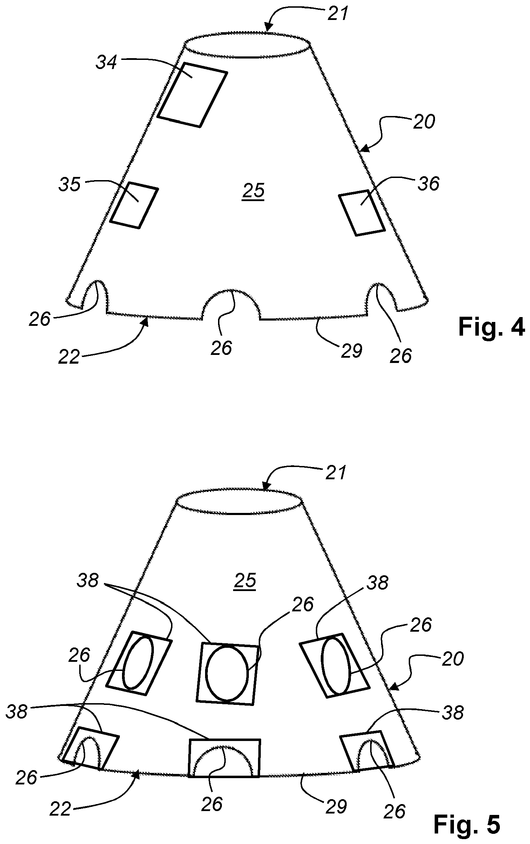

**Figur 4** zeigt eine perspektivische Ansicht einer weiteren Ausführungsform des Expansionselements 20. Hier sind neben dem RFID-Chip 34 und der Mantelfläche 25 des Expansionselements 20 noch ein Farbsensor 35 und ein Ozon-Sensor 36 zugeordnet. Mit dem Farbsensor 35 kann angezeigt werden, dass die Behandlung des Wundbereichs 13 abgeschlossen ist. Es ist selbstverständlich, dass der RFID-Chip 34, der Farbsensor 35 und der Ozon-Sensor 36 allein und/oder in beliebiger Kombination der Mantelfläche 25 des Expansionselements 20 zugeordnet sein können. Das hier dargestellte Expansionselement 20 hat am umlaufenden Rand 29 mehrere Durchbrüche 26 ausgebildet.

**Figur 5** zeigt eine weitere mögliche Ausführungsform des Expansionselements 20. Das hier dargestellte Expansionselement 20 hat nicht nur am umlaufenden Rand 29 mehrere Durchbrüche 26 ausgebildet sondern auch auf der Mantelfläche 25. Jeder Durchbruch 26 ist mit einem Filter 38 abgedeckt, so dass nur die Gasbestandteile austreten können, die man aus dem Innern des Expansionselements 20 entfernen will und die man ggf. einer erneuten Verwendung zuführen möchte.

Es ist besonders vorteilhaft, wenn das Expansionselement 20 als Single-Use-Element ausgebildet ist. Ein Desinfektionsvorgang kann vermieden werden, da das Expansionselement 20 nach jedem Gebrauch entsorgt wird. Das Expansionselement 20 kann auf kostengünstige Weise mit einem Spritzgussprozess oder Tiefziehprozess oder durch Thermoumformen hergestellt werden. Es ist selbstverständlich, dass das Expansionselement 20 in verschiedenen Ausstattungsvarianten zur Verfügung gestellt werden kann. Bei einer maximalen Ausstattung hat das Expansionselement 20 mehrere Durchbrüche 26, die jeweils mit einem Filter 38 versehen sind. Hinzu kommen der RFID-Chip 34, der Farbsensor 35 und der Ozon-Sensor 36. Für einen Fachmann ist es selbstverständlich, dass die maximale Ausstattung keine Beschränkung der Erfindung darstellt.

### Bezugszeichenliste:

- 1: Anordnung
- 3: Steuerschaltung
- 4: elektronische Bauelemente
- 5: piezoelektrischer Transformator
- 6: Gasleitung
- 7: Platine
- 8: Hochspannungsende
- 10: Plasmaerzeugungsgerät
- 11: umlaufender Rand
- 12: elektrischer Anschluss
- 13: Wundbereich
- 14: Strömungsleitelement
- 15: Plasmaströmung
- 17: Lüfter
- 18: Querschnittsfläche des ersten Endes
- 19: Querschnittsfläche des zweiten Endes
- 20: Expansionselement
- 21: erstes Ende
- 22: zweites Ende
- 23: Kabel von Netzteil
- 24: Seite des piezoelektrischen Transformator
- 25: Mantelfläche
- 26: Durchbrüche
- 27: Innenfläche
- 28: Rückführung
- 29: umlaufender Rand
- 30: Gehäuse
- 32: Öffnung
- 34: RFID-Chip
- 35: Farbsensor
- 36: Ozon-Sensor
- 38: Filter
- 39: Pumpe
- 40: Körperteil
- H: Höhe
- P: Plasma

## Patentansprüche

1. Anordnung (1) zur Behandlung von Wunden umfassend:
• ein Gerät (10) zur Erzeugung eines Plasmas (P) und/oder eines angeregten Gases bzw. Gasgemisches, wobei ein piezoelektrischer Transformator (5) zusammen mit einer Platine (7) in einem Gehäuse (30) des Gerätes (10) untergebracht ist, wobei auf der Platine (7) eine Steuerschaltung (3) realisiert ist, mit der der piezoelektrische Transformator (5) elektrisch verbunden ist, wobei der Transformator und die Platine inklusive Steuerschaltung Bestandteile des Geräts sind und wobei eine Öffnung (32) des Gehäuses (30) zu einem Hochspannungsende (8) des piezoelektrischen Transformators (5) hin ausgerichtet ist;
• einen Lüfter (17), der im oder am Gehäuse (30) vorgesehen ist und im Gehäuse (30) für eine Luftströmung zur Öffnung (32) des Gehäuses (30) hin sorgt, wobei das mit dem Gerät (10) erzeugte Plasma (P) und/oder das angeregte Gas bzw. Gasgemisch aus der Öffnung (32) des Gehäuses (30) austritt; und
• ein Expansionselement (20), das mit einem ersten Ende (21) lösbar mit dem Gehäuse (30) des Geräts (10) an dessen Öffnung (32) verbunden ist und mit einem zweiten Ende (22) einen zu behandelnden Wundbereich (13) umgibt, wobei eine Querschnittsfläche (19) des zweiten Endes (22) des Expansionselements (20) größer ist als eine Querschnittsfläche (18) des ersten Endes (21) des Expansionselements (20).

2. Anordnung (1) nach Anspruch 1, wobei eine Mantelfläche (25) des Expansionselements (20) mehrere Durchbrüche (26) ausgebildet hat.

3. Anordnung (1) nach Anspruch 2, wobei an einer Innenfläche (27) der Mantelfläche (25) mindestens ein Strömungsleitelement (14) angebracht ist, damit eine Strömung (15) aus Plasma (P) oder angeregtem Gas bzw. Gasgemisch auf den zu behandelnden Wundbereich (13) richtbar ist.

4. Anordnung (1) nach den vorangehenden Ansprüchen, wobei eine Rückführung (28) das Expansionselement (20) und das Gerät (10) verbindet.

5. Anordnung (1) nach den vorangehenden Ansprüchen, wobei ein Farbsensor (35) dem Expansionselement (20) zugeordnet ist, um über eine Farbänderung anzuzeigen, dass die Behandlung abgeschlossen ist.

6. Anordnung (1) nach den vorangehenden Ansprüchen, wobei ein RFID-Chip (34) dem Expansionselement (20) zugeordnet ist, so dass über den Typ des Expansionselements (20) und in Verbindung mit dem Gerät (10) zumindest eine Behandlungsdauer einstellbar ist.

7. Anordnung (1) nach den vorangehenden Ansprüchen, wobei ein Ozon-Sensor (36) dem Expansionselement (20) zugeordnet ist.

8. Anordnung (1) nach den vorangehenden Ansprüchen, wobei je ein Filter (38) je einem Durchbruch (26) des Expansionselements (20) zugeordnet ist.

9. Anordnung (1) nach den Ansprüchen 1 bis 8, wobei das Gerät (10) ein Handgerät ist.

## Claims

1. Assembly (1) for the treatment of wounds comprising:
• a device (10) for generating a plasma (P) and/or an excited gas or gas mixture, wherein a piezoelectric transformer (5) is housed together with a circuit board (7) in a housing (30) of the device (10), wherein a control circuit (3) is realized on the circuit board (7), to which control circuit (3) the piezoelectric transformer (5) is electrically connected, wherein the transformer and the circuit board, including the control circuit, are components of the device and wherein an opening (32) of the housing (30) is aligned to a high voltage end (8) of the piezoelectric transformer (5);
• a fan (17) which is provided in or on the housing (30) and provides in the housing (30) an airflow to the opening (32) of the housing (30), wherein the plasma (P) generated by the device (10) and/or the excited gas or gas mixture emerges from the opening (32) of the housing (30); and
• an expansion element (20), which is releasably connected with a first end (21) to the housing (30) of the device (10) at the opening (32) thereof and surrounds a wound area to be treated (13) with a second end (22), wherein a cross-sectional area (19) of the second end (22) of the expansion element (20) is greater than a cross-sectional area (18) of the first end (21) of the expansion element (20).

2. Assembly (1) according to claim 1, wherein a lateral surface (25) of the expansion element (20) forms a plurality of openings (26).

3. Assembly (1) according to claim 2, wherein on an inner surface (27) of the lateral surface (25) at least one flow guide element (14) is mounted so that a flow (15) of plasma (P) or excited gas or gas mixture can be directed to the wound area to be treated (13).

4. Assembly (1) according to anyone of the preceding claims, wherein a return (28) connects the expansion element (20) and the device (10).

5. Assembly (1) according to anyone of the preceding claims, wherein a color sensor (35) is associated with the expansion element (20) to indicate via a color change that the treatment is completed.

6. Assembly (1) according to anyone of the preceding claims, wherein an RFID chip (34) is associated with the expansion element (20), so that at least one treatment time is adjustable via the type of the expansion element (20) and in connection with the device (10).

7. Assembly (1) according to anyone of the preceding claims, wherein an ozone sensor (36) is associated with the expansion element (20).

8. Assembly (1) according to anyone of the preceding claims, wherein one filter (38) each is associated with a respective opening (26) of the expansion element (20).

9. Assembly (1) according to anyone of claims 1 to 8, wherein the device (10) is a hand-held device.

## Revendications

1. Dispositif (1) pour le traitement de plaies, comprenant :
• un appareil (10) destiné à produire un plasma (P) et/ou un gaz ou un mélange de gaz excité, un transformateur piézoélectrique (5) étant logé avec une carte de circuit imprimé (7) dans un boîtier (30) de l'appareil (10), un circuit de commande (3) étant réalisé sur la carte de circuit imprimé (7), auquel le transformateur piézoélectrique (5) est relié électriquement, le transformateur et la carte de circuit imprimé incluant le circuit de commande étant des éléments constitutifs de l'appareil et une ouverture (32) du boîtier (30) étant dirigée vers une extrémité haute tension (8) du transformateur piézoélectrique (5) ;
• un ventilateur (17) qui est prévu dans ou sur le boîtier (30) et assure dans le boîtier (30) un écoulement d'air vers l'ouverture (32) du boîtier (30), le plasma (P) et/ou le gaz ou mélange de gaz excité produit avec l'appareil (10) sortant par l'ouverture (32) du boîtier (30) ; et
• un élément d'expansion (20) qui est relié de manière amovible par une première extrémité (21) au boîtier (30) de l'appareil (10) à son ouverture (32) et entoure par une deuxième extrémité (22) une zone de plaie (13) à traiter, une surface de section transversale (19) de la deuxième extrémité (22) de l'élément d'expansion (20) étant plus grande qu'une surface de section transversale (18) de la première extrémité (21) de l'élément d'expansion (20).

2. Dispositif (1) selon la revendication 1, dans lequel une surface latérale (25) de l'élément d'expansion (20) présente plusieurs ouvertures (26).

3. Dispositif (1) selon la revendication 2, dans lequel au moins un élément de guidage d'écoulement (14) est monté sur une surface intérieure (27) de la surface latérale (25), de sorte qu'un écoulement (15) de plasma (P) ou de gaz ou mélange de gaz excité peut être dirigé sur la zone de plaie (13) à traiter.

4. Dispositif (1) selon l'une des revendications précédentes, dans lequel une contre-réaction (28) relie l'élément d'expansion (20) et l'appareil (10).

5. Dispositif (1) selon l'une des revendications précédentes, dans lequel un capteur de couleur (35) est associé à l'élément d'expansion (20) pour indiquer par un changement de couleur que le traitement est terminé.

6. Dispositif (1) selon l'une des revendications précédentes, dans lequel une puce RFID (34) est associée à l'élément d'expansion (20), de sorte que, par le type de l'élément d'expansion (20) et en liaison avec l'appareil (10), au moins une durée de traitement peut être réglée.

7. Dispositif (1) selon l'une des revendications précédentes, dans lequel un capteur d'ozone (36) est associé à l'élément d'expansion (20).

8. Dispositif (1) selon l'une des revendications précédentes, dans lequel un filtre (38) est associé à chaque ouverture (26) de l'élément d'expansion (20).

9. Dispositif (1) selon l'une des revendications 1 à 8, dans lequel l'appareil (10) est un appareil portatif.
